# FASCICULE DE BREVET EUROPEEN

(11) **EP 3 600 455 B1**
(45) Date de publication et mention de la délivrance du brevet: **13.08.2025**
(21) Numéro de dépôt: 18719053.3
(22) Date de dépôt: 20.03.2018
(51) Int. Cl.: A61L 2/07, B27K 5/00, A61L 2/00

(54) **DISPOSITIF DE TRAITEMENT THERMIQUE DE GRUMES**
VORRICHTUNG ZUR WÄRMEBEHANDLUNG VON HOLZSTÄMMEN
DEVICE FOR THE THERMAL TREATMENT OF LOGS

(30) Priorité: 20.03.2017 FR 1770267
(43) Date de publication de la demande: 05.02.2020
(73) Titulaire: Thermodynamic Workshop Training - TWT, 76600 Le Havre (FR)
(72) Inventeur: PINAULT, Philippe, 35150 Amanlis (FR); PINAULT, Valentin, 35150 Amanlis (FR); GATT, Anthony-Ange, 76600 Le Havre (FR)
(74) Mandataire: Hege, Frédéric
(86) Numéro de dépôt international: PCT/EP2018/056998
(87) Numéro de publication internationale: WO 2018/172343

(56) Documents cités:
- WO-A1-2015/081227
- WO-A1-90/06840
- WO-A1-95/35191
- GB-A- 253 041
- RU-C1- 2 545 030
- SU-A1- 1 652 618
- US-A- 80 754
- US-A1- 2012 247 067

## Description

La présente invention se situe dans le domaine du traitement phytosanitaire des grumes, en particulier de feuillus et résineux. Elle concerne plus particulièrement dispositif de traitement thermique sous écorce de grumes.

Le transport de grumes participe à la dissémination d'organismes nuisibles aux végétaux, notamment des champignons et des insectes. Il est donc nécessaire de traiter les grumes, afin de limiter ce phénomène. Un tel traitement phytosanitaire est même obligatoire pour l'exportation de grumes vers certains pays. Il consiste selon les normes actuelles à maintenir sous écorce (sous cortical) l'ensemble des grumes à une température supérieure à 71,1°C pendant au moins 75 minutes. Il est ainsi habituellement procédé à un traitement thermique, consistant en l'injection dans un conteneur de grumes de flux d'air chaud ou de vapeur, à une température comprise entre 80 et 150°C. L'injection est réalisée depuis l'arrière du conteneur, après avoir ouvert les portes. La chaleur venant de l'arrière du conteneur, ce procédé ne permet pas une répartition homogène de la chaleur dans le conteneur, et résulte en une consommation d'énergie importante. Le cycle du traitement thermique est également long du fait d'un important temps de montée en température sous écorce des grumes les plus éloignées de la source de chaleur.

Le document US5447686 divulgue un procédé de traitement thermique de pièces de bois par de la vapeur pour y supprimer les différents parasites. Les pièces de bois sont chargées dans la cale d'un bateau équipé pour la production de vapeur. Lesdites pièces sont mises en contact avec la vapeur, injectée par le fond de la cale, pour en élever la température et la maintenir suffisamment longtemps à un niveau suffisant pour supprimer les différents parasites pouvant être présents. Cependant pour appliquer ce procédé, le bois ne peut pas rester dans un conteneur mais doit être déchargé dans la cale du bateau.

Le document US20122147067 divulgue un dispositif de réchauffage d'une charge liquide dans un conteneur, mais la solution ne convient pas pour le traitement de pièces solides.

La présente invention a pour objet de pallier au moins en partie à ces inconvénients en proposant un dispositif permettant de traiter des grumes se trouvant dans un conteneur sans avoir sortir lesdites grumes dudit conteneur. L'opération peut avoir lieu dans le conteneur resté sur la semi-remorque attelée à son tracteur, sans aucune manipulation des grumes. Le dispositif selon l'invention permet également une montée en température rapide des grumes en tout point du conteneur.

A cet effet, la présente invention propose un dispositif de traitement thermique comprenant un conteneur chargé de grumes, et une conduite d'arrivée de vapeur d'eau sous pression. Ledit dispositif de traitement thermique est particulier en ce qu'il comporte au moins un, de préférence deux, tube muni d'une pluralité d'ouvertures réparties sur sa longueur et sur sa circonférence, ledit au moins un tube étant destiné à être introduit depuis l'arrière dans ledit conteneur chargé de grumes, et à être alimenté par ladite vapeur d'eau sous pression de sorte que la vapeur d'eau vienne au contact des grumes, ledit au moins un tube étant sensiblement rectiligne dans le sens qu'il présente une certaine élasticité, lui permettant de contourner une grume pour trouver son chemin vers l'avant du conteneur.

Grâce à ces dispositions, l'injection de chaleur étant répartie sur toute la longueur du conteneur, le traitement thermique des grumes est rapide, et optimal en termes de consommation d'énergie. Le dispositif permet aussi un traitement thermique facile à mettre en œuvre dans un conteneur standard tel qu'utilisé pour le transport par camion ou bateau.

Selon d'autres caractéristiques :
- lesdites ouvertures desdits tubes sont de dimension et/ou nombre croissant en partant du côté de l'alimentation en vapeur d'eau, améliorant la répartition de la vapeur dans le conteneur afin que les grumes situées à l'extrémité du conteneur opposée à l'alimentation en vapeur des tubes, et convoyant donc de la vapeur d'avantage refroidie où déjà condensée, puissent monter en température aussi rapidement que les grumes situées proche de l'alimentation,
- ledit au moins un tube peut présenter un diamètre extérieur inférieur à 25 mm, ce qui permet de facilement l'insérer dans le conteneur dans les espaces libres entre les grumes,
- ledit dispositif peut comporter en outre une gouttière de récupération d'eau condensée, permettant l'évacuation et la récupération de l'eau produite au cours du traitement thermique par la condensation de la vapeur pour un autre usage,
- ledit dispositif peut comporter en outre une conduite d'évacuation de l'eau récupérée dans la gouttière, ladite conduite étant munie d'un filtre, permettant de récupérer l'eau de la gouttière pour un autre usage, par exemple la production de vapeur d'eau pour alimenter la conduite,
- ledit dispositif peut comporter en outre un dispositif d'étanchéité, consistant en particulier en au moins une bande de mousse, destiné à être disposé au niveau de l'ouverture arrière dudit conteneur en vue de réduire l'échappement de vapeur d'eau et de chaleur, permettant de réduire la consommation d'énergie du dispositif selon l'invention ; en particulier on peut refermer la porte de gauche après mise en place du dispositif, et disposer ladite bande de mousse au niveau de l'ouverture de la porte de droite,
- ladite bande de mousse peut être munie d'une bande magnétique pour sa fixation au conteneur, ce qui constitue un mode de réalisation simple et facile à mettre en œuvre, même de façon répétée, de l'invention.

La présente invention concerne également un procédé de traitement thermique de grumes chargées dans un conteneur, caractérisé en ce qu'il comporte les étapes suivantes :
- ouverture du conteneur par l'arrière,
- introduction d'au moins un tube, depuis l'arrière dans ledit conteneur chargé de grumes, ledit tube étant muni d'une pluralité d'ouvertures réparties sur sa longueur et sur sa circonférence, de sorte qu'il s'étende sur au moins les trois quarts de la longueur du conteneur, ledit au moins un tube étant sensiblement rectiligne dans le sens qu'il présente une certaine élasticité, lui permettant de contourner une grume pour trouver son chemin vers l'avant du conteneur,
- raccordement dudit tube à une conduite d'alimentation en vapeur d'eau,
- mise sous pression de la conduite.

On peut, avant la mise sous pression de la conduite, refermer la porte de gauche du conteneur, puis bloquer la porte de droite entrouverte.

Grâce à ces dispositions, l'injection de chaleur étant répartie sur toute la longueur du conteneur, le traitement thermique des grumes est rapide, optimal en termes de consommation d'énergie. Un tel procédé de traitement thermique est facile à mettre en œuvre dans un conteneur standard tel qu'utilisé pour le transport par camion ou bateau.

Selon d'autres caractéristiques :
- ledit procédé peut comporter en outre une étape d'étanchéification des ouvertures de porte par un dispositif d'étanchéité avant la mise sous pression de la conduite, permettant de réduire la consommation d'énergie du procédé selon l'invention.

La présente invention sera mieux comprise à la lecture de la description détaillée qui fait suite, en référence aux figures annexées dans lesquelles :

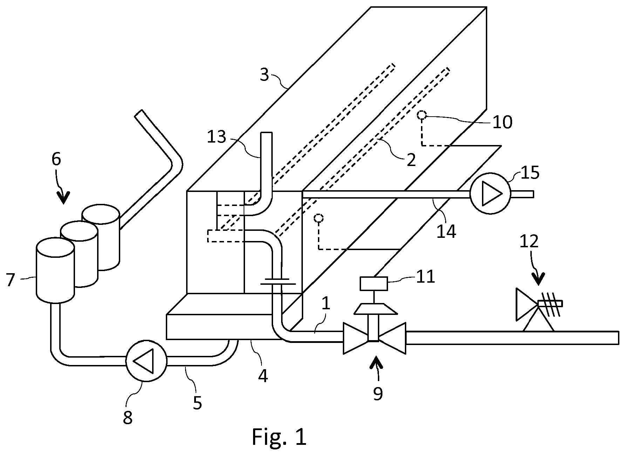

- La figure 1 est une vue schématique du dispositif de traitement thermique selon l'invention.

Le dispositif de traitement thermique selon l'invention, illustré en fig. 1, comporte une conduite 1 d'arrivée de vapeur d'eau sous pression, alimentant un tube 2 destiné à être introduit dans un conteneur 3 de grumes par l'arrière.

Ce dispositif est destiné à être introduit dans un conteneur 3, il est donc dissociable dudit conteneur 3, et peut servir à traiter plusieurs conteneurs 3 distincts l'un après l'autre.

Le tube 2 est sensiblement rectiligne, et il est muni d'une pluralité d'ouvertures réparties sur sa longueur et sur sa circonférence. Ces ouvertures peuvent consister en des entailles sciées dans le tube 2. Le tube 2 a une longueur qui peut être par exemple d'environ 10 mètres, afin qu'une fois introduit dans le conteneur 3, la vapeur d'eau s'échappant par ses ouvertures puisse rapidement atteindre l'ensemble des grumes du conteneur 3 et sur toute leur longueur. Le traitement thermique peut ainsi être homogène dans l'ensemble du conteneur 3. Afin d'obtenir un résultat optimal, le tube 2 peut être inséré à mi-hauteur dans le conteneur 3.

Dans le mode de réalisation préféré de l'invention illustré en fig. 1, le dispositif de traitement thermique selon l'invention comporte deux tubes 2, afin d'améliorer encore la répartition de la vapeur d'eau dans le conteneur 3. Les deux tubes 2 peuvent par exemple être placés tous deux à mi-hauteur du conteneur 3 et répartis sur sa largeur.

Les ouvertures du tube 2 peuvent être de dimension et/ou de nombre croissant à partir de l'extrémité alimentée par la conduite 1. Ceci permet de s'assurer que suffisamment de vapeur d'eau atteigne l'extrémité du tube 2 la plus éloignée de l'alimentation en vapeur d'eau pour obtenir un environnement thermique uniforme au sein du conteneur 3.

Le tube 2 peut présenter un diamètre extérieur inférieur à 25 mm, afin de pouvoir être inséré facilement parmi les grumes dans le conteneur 3. Cependant ce diamètre extérieur doit être suffisamment important pour transporter la quantité voulue de vapeur d'eau. L'épaisseur du tube 2 peut être d'au moins 2 mm, afin que le tube 2 soit suffisamment solide et ne se déforme pas trop lorsqu'on l'insère entre les grumes du conteneur 3, tout en résistant à la pression exercée en son sein par la vapeur d'eau. Le tube 2 peut présenter une certaine élasticité, lui permettant par exemple de contourner une grume pour trouver son chemin vers l'avant du conteneur. Une telle élasticité fera que le tube sera légèrement déformé, mais on dans le cadre de la présente invention il doit néanmoins être considéré comme étant « sensiblement rectiligne ».

Une condensation partielle de la vapeur a lieu dans le tube 2, libérant ainsi la majeure partie de la chaleur latente sans endommager les grumes. Le reste de la vapeur se condense au contact de l'écorce des grumes, garantissant une montée en température optimale de ces dernières.

Le dispositif selon l'invention peut encore comporter un dispositif d'étanchéité, consistant par exemple en une ou plusieurs bandes de mousse. Le dispositif d'étanchéité peut comporter des bandes magnétiques pour sa fixation sur le conteneur, et il peut être disposé au niveau de l'ouverture arrière du conteneur, en particulier au niveau de l'ouverture de la porte droite par laquelle est insérée l'extrémité de la conduite 1 et le tube 2. Ceci permet de réduire l'échappement de vapeur d'eau et de chaleur du conteneur 3 pendant le traitement thermique, et donc de réduire la consommation d'énergie du dispositif de traitement thermique.

Dans le mode de réalisation de la fig. 1, le dispositif de traitement thermique comporte une gouttière 4 de récupération d'eau, sous la forme d'un bac placé à l'arrière du conteneur 3. On peut alors incliner le conteneur 3, par exemple d'un angle de l'ordre de 5 à 10 degrés, afin que l'eau condensée et les éventuels tanins produits lors du traitement thermique s'écoulent vers la gouttière 4.

L'eau récupérée dans la gouttière 4 peut ensuite être dirigée vers une conduite d'évacuation 5 qui peut comporter un filtre 6. Le filtre 6 peut comprendre un ou plusieurs bacs 7 de filtrage, chaque bac comprenant une ou plusieurs couches de matériau filtrant tel que de la mousse filtrante, du charbon actif et de l'ouate filtrante fine. L'eau évacuée peut être envoyée, par exemple grâce à une pompe 8 à membrane, de la gouttière 4 vers le filtre 6 et parcourir successivement les différentes couches des bacs 7. L'eau filtrée peut ensuite être récupérée pour tout usage, par exemple vers la chaudière produisant la vapeur d'eau de la conduite 1. Cette chaudière peut être une chaudière brûlant du déchet, permettant ainsi une très faible consommation de ressources naturelles.

Un tuyau 14 peut être disposé au niveau de la porte droite dans le coin de la charnière haute du conteneur pour de tirer au vide l'enceinte du conteneur. Ainsi on peut extraire l'air froid en début de traitement et créer une circulation à la demande.

Un tel tirage au vide (sous légère pression atmosphérique) peut être assuré par une pompe à vide ou un éjecteur d'air vapeur 15. Il peut être manœuvré par une vanne à la demande.

Pour réduire le temps de montée en température, et/ou pour réduire la consommation de vapeur, on peut disposer un isolant, par exemple de la laine de verre, à l'intérieur sur les parois du conteneur, et/ou disposer une couverture sur le dessus des grumes, par exemple de type film pare vapeur tel que ceux utilisés comme isolant de toiture.

On peut aussi ajouter un tube 2 sur le dessus des grumes, le cas échéant sous ladite couverture, pour optimiser l'accès aux grumes de la vapeur.

Le procédé de traitement thermique selon la demande est un traitement de grumes directement dans le conteneur dans lequel elles sont chargées. Il comporte les étapes suivantes :
- ouverture du conteneur 3 par l'arrière, par exemple une des portes du conteneur 3 peut être entrouverte.
- introduction d'au moins un tube 2 depuis l'arrière dans ledit conteneur chargé de grumes, ledit tube 2 étant muni d'une pluralité d'ouvertures réparties sur sa longueur et sur sa circonférence, de sorte qu'il s'étende sur au moins les trois quarts de la longueur du conteneur, de préférence sensiblement sur toute la longueur du conteneur 3 ledit au moins un tube 2 étant sensiblement rectiligne dans le sens qu'il présente une certaine élasticité, lui permettant de contourner une grume pour trouver son chemin vers l'avant du conteneur ; par exemple si le conteneur fait une longueur de 12 m, un tube de 10 m peut être utilisé,
- raccordement dudit tube 2 à une conduite 1 d'alimentation en vapeur d'eau,
- mise sous pression de la conduite 1, par exemple une pression au moins égale à 5 bar.

Une étape d'étanchéification du conteneur 3 peut être ajoutée au précédent procédé juste avant la mise sous pression de la conduite 1, afin que la vapeur sous pression issue de la conduite 1 et répandue dans le conteneur 3 par l'intermédiaire du tube 2 ne s'échappe pas en grande partie par l'ouverture du conteneur 3.

Par exemple, l'une des deux portes arrière est maintenue fermée, l'autre est entr'ouverte pour laisser passer la conduite 1 de vapeur d'eau et les tubes 2. L'entr'ouverture de cette porte crée un espace d'ouverture, de largeur environ 20 mm et de la hauteur de la porte. Cet espace peut alors être comblé par une bande d'une mousse polymère résistant aux conditions de température et de pression régnant dans le conteneur. Selon la disposition de la porte par rapport au plafond et plancher du conteneur, on pourra mettre en place une telle bande de mousse également sur le dessus et/ou le dessous de la porte entr'ouverte.

On peut prévoir de disposer sur la bande de mousse une bande magnétique, permettant la fixation de la bande de mousse sur le conteneur, et son retrait facile après le traitement, pour pouvoir être réutilisé pour un autre traitement.

La conduite 1 d'alimentation en vapeur d'eau peut comporter un détendeur 9. Ce dernier permet de réguler l'apport de vapeur dans le conteneur 3 durant le traitement thermique. Le dispositif peut alors comporter des capteurs de température 10, placés à certains endroits du conteneur 3, et un calculateur 11, afin d'automatiser la commande du détendeur 9 et d'asservir en température l'apport de vapeur dans le conteneur 3.

En amont du détendeur 9, la conduite 1 peut comporter une soupape 12 de sécurité. En cas de surpression dans la conduite 1 en amont du détendeur, la soupape 12 peut faire descendre la pression pour éviter de mettre en danger le personnel situé aux alentours. La soupape 12 peut être tarée par exemple à 10 bar.

Le dispositif peut encore comporter un dispositif de sécurité 13 de type « mast riser » contrôlant la pression au sein du conteneur 3 afin d'éviter une surpression pendant le traitement thermique. Le dispositif de sécurité 13 peut aussi consister simplement en une interruption de la bande de mousse pour laisser s'échapper le surplus de vapeur, cette interruption étant correctement dimensionnée pour maintenir la pression souhaitée dans le conteneur, sans laisser la pression monter au-delà de l'acceptable.

Selon un autre mode de réalisation, on dispose au moins une sonde de température dans l'enceinte du conteneur, en un point judicieusement choisi pour donner une indication sur la température la plus basse dans les grumes, ce qui permet de surveiller que sous écorce tous les points des grumes se trouvent bien au-dessus de la température prescrite. Le pilotage du détendeur, en augmentant la pression pour monter la température et en la diminuant pour baisser la température, peut se faire automatiquement, mais aussi manuellement, grâce à un simple affichage de la température mesurée.

Bien que la description ci-dessus se base sur des modes de réalisation particuliers, elle n'est nullement limitative de la portée de l'invention, et des modifications peuvent être apportées, notamment par substitution d'équivalents techniques ou par combinaison différente de tout ou partie des caractéristiques développées ci-dessus.

## Revendications

1. Dispositif de traitement thermique comprenant un conteneur (3) chargé de grumes, et une conduite (1) d'arrivée de vapeur d'eau sous pression, **caractérisé en ce qu'**il comporte au moins un, de préférence deux, tube (2) muni d'une pluralité d'ouvertures réparties sur sa longueur et sur sa circonférence, ledit au moins un tube (2) étant destiné à être introduit depuis l'arrière dans ledit conteneur (3) chargé de grumes, et à être alimenté par ladite vapeur d'eau sous pression de sorte que la vapeur d'eau vienne au contact des grumes, ledit au moins un tube (2) étant sensiblement rectiligne dans le sens qu'il présente une certaine élasticité, lui permettant de contourner une grume pour trouver son chemin vers l'avant du conteneur (3).

2. Dispositif selon la revendication précédente, dans lequel lesdites ouvertures desdits tubes (2) sont de dimension et/ou nombre croissant en partant du côté de l'alimentation en vapeur d'eau.

3. Dispositif selon l'une des revendications précédentes, dans lequel ledit au moins un tube (2) présente un diamètre extérieur inférieur à 25 mm.

4. Dispositif selon l'une des revendications précédentes, comportant en outre une gouttière (4) de récupération d'eau condensée.

5. Dispositif selon la revendication précédente, comportant en outre une conduite (5) d'évacuation de l'eau récupérée dans la gouttière (4), ladite conduite (5) étant munie d'un filtre (6).

6. Dispositif selon l'une des revendications précédentes, comportant en outre un dispositif d'étanchéité, consistant en particulier en au moins une bande de mousse, destiné à être disposé au niveau de l'ouverture arrière dudit conteneur (3) en vue de réduire l'échappement de vapeur d'eau et de chaleur.

7. Dispositif selon la revendication précédente, dans lequel ladite bande de mousse est munie d'une bande magnétique pour sa fixation au conteneur (3).

8. Procédé de mise en œuvre d'un dispositif selon l'une des revendications précédentes pour le traitement thermique de grumes chargées dans un conteneur (3), **caractérisé en ce qu'**il comporte les étapes suivantes :
- ouverture du conteneur (3) par l'arrière,
- introduction d'au moins un tube (2) depuis l'arrière dans ledit conteneur (3) chargé de grumes, ledit tube (2) étant muni d'une pluralité d'ouvertures réparties sur sa longueur et sur sa circonférence, de sorte qu'il s'étende sur au moins les trois quarts de la longueur du conteneur, ledit au moins un tube (2) étant sensiblement rectiligne dans le sens qu'il présente une certaine élasticité, lui permettant de contourner une grume pour trouver son chemin vers l'avant du conteneur (3)
- raccordement dudit tube (2) à une conduite (1) d'alimentation en vapeur d'eau,
- mise sous pression de la conduite (1).

9. Procédé selon la revendication précédente, comportant en outre une étape d'étanchéification des ouvertures de porte par un dispositif d'étanchéité avant la mise sous pression de la conduite (1).

10. Procédé selon la revendication 8 dans lequel l'introduction d'au moins un tube (2) se fait de sorte qu'il s'étende sur toute la longueur du conteneur.

## Patentansprüche

1. Vorrichtung zur Wärmebehandlung mit einem mit Stämmen beladenen Behälter (3) und einer Leitung (1) zur Zufuhr von unter Druck stehendem Wasserdampf, **dadurch gekennzeichnet, dass** sie mindestens ein, vorzugsweise zwei, Rohr (2) mit einer Vielzahl von Öffnungen aufweist, die über ihre Länge und ihren Umfang verteilt sind, wobei das mindestens eine Rohr (2) dazu bestimmt ist, von hinten in den mit Stämmen beladenen Behälter (3) eingeführt zu werden, und mit dem Wasserdampf unter Druck versorgt zu werden, so dass der Wasserdampf mit den Stämmen in Kontakt kommt, wobei das mindestens eine Rohr (2) im Wesentlichen geradlinig in dem Sinne ist, dass es eine gewisse Elastizität aufweist, die es ihm ermöglicht, einen Stamm zu umgehen, um seinen Weg nach vorne in den Behälter (3) zu finden.

2. Vorrichtung nach dem vorhergehenden Anspruch, wobei die Öffnungen der Rohre (2) von der Seite der Wasserdampfzufuhr aus gesehen eine zunehmende Größe und/oder Anzahl aufweisen.

3. Vorrichtung nach einem der vorhergehenden Ansprüche, wobei das mindestens eine Rohr (2) einen Außendurchmesser von weniger als 25 mm aufweist.

4. Vorrichtung nach einem der vorhergehenden Ansprüche, die außerdem eine Rinne (4) zum Auffangen von kondensiertem Wasser umfasst.

5. Vorrichtung nach dem vorhergehenden Anspruch, die außerdem eine Leitung (5) zum Ableiten des in der Rinne (4) gesammelten Wassers umfasst, wobei die Leitung (5) mit einem Filter (6) versehen ist.

6. Vorrichtung nach einem der vorhergehenden Ansprüche, die außerdem eine Dichtungsvorrichtung umfasst, die insbesondere aus mindestens einem Schaumstoffstreifen besteht und dazu bestimmt ist, an der hinteren Öffnung des Behälters (3) angeordnet zu werden, um das Entweichen von Wasserdampf und Wärme zu reduzieren.

7. Vorrichtung nach dem vorhergehenden Anspruch, wobei der Schaumstoffstreifen mit einem Magnetstreifen zu seiner Befestigung am Behälter (3) versehen ist.

8. Verfahren zum Betreiben einer Vorrichtung nach einem der vorhergehenden Ansprüche zur Wärmebehandlung von in einem Behälter (3) geladenen Stämmen, **dadurch gekennzeichnet, dass** es die folgenden Schritte umfasst:
- Öffnen des Behälters (3) von hinten,
- Einführen mindestens eines Rohrs (2) von hinten in den mit Stämmen beladenen Behälter (3), wobei das Rohr (2) mit einer Vielzahl von Öffnungen versehen ist, die über seine Länge und seinen Umfang verteilt sind, so dass es sich über mindestens drei Viertel der Länge des Behälters erstreckt, wobei das mindestens eine Rohr (2) im Wesentlichen geradlinig in dem Sinne ist, dass es eine gewisse Elastizität aufweist, die es ihm ermöglicht, einen Stamm zu umfahren, um seinen Weg zur Vorderseite des Behälters (3) zu finden.
- Anschluss des genannten Rohrs (2) an eine Leitung (1) zur Versorgung mit Wasserdampf,
- Druckbeaufschlagung der Leitung (1).

9. Verfahren nach dem vorhergehenden Anspruch, das außerdem einen Schritt des Abdichtens der Türöffnungen durch eine Dichtungsvorrichtung umfasst, bevor die Leitung (1) unter Druck gesetzt wird.

10. Verfahren nach Anspruch 8, bei dem die Einführung mindestens eines Rohrs (2) so erfolgt, dass es sich über die gesamte Länge des Behälters erstreckt.

## Claims

1. Heat treatment device comprising a container (3) loaded with logs, and a pipe (1) for supplying pressurised steam, **characterised in that** it comprises at least one, preferably two, tubes (2) provided with a plurality of openings distributed along its length and around its circumference, the said at least one tube (2) being intended to be introduced from the rear into the said container (3) loaded with logs, and to be supplied with said steam under pressure so that the steam comes into contact with the logs, said at least one tube (2) being substantially straight in the sense that it has a certain elasticity, enabling it to go around a log to find its way towards the front of the container (3).

2. Device according to the preceding claim, wherein said openings of said tubes (2) are of increasing size and/or number starting from the steam supply side.

3. Device according to one of the preceding claims, wherein said at least one tube (2) has an external diameter of less than 25 mm.

4. Device according to one of the preceding claims, further comprising a gutter (4) for collecting condensed water.

5. Device according to the preceding claim, further comprising a pipe (5) for draining the water recovered in the gutter (4), said pipe (5) being provided with a filter (6).

6. Device according to one of the preceding claims, further comprising a sealing device, consisting in particular of at least one strip of foam, intended to be arranged at the rear opening of the said container (3) with a view to reducing the escape of water vapour and heat.

7. Device according to the preceding claim, in which the said foam strip is provided with a magnetic strip for fixing it to the container (3).

8. Method of using a device according to one of the preceding claims for the heat treatment of logs loaded into a container (3), **characterised in that** it comprises the following steps:
- opening the container (3) from the rear,
- introducing at least one tube (2) from the rear into the said container (3) loaded with logs, the said tube (2) being provided with a plurality of openings distributed over its length and circumference, so that it extends over at least three quarters of the length of the container, the said at least one tube (2) being substantially straight in the sense that it has a certain elasticity, enabling it to go around a log to find its way towards the front of the container (3).
- connecting the said tube (2) to a steam supply pipe (1),
- pressurising the pipe (1).

9. Method according to the preceding claim, further comprising a step of sealing the door openings with a sealing device before pressurising the pipe (1).

10. Method according to claim 8, in which at least one tube (2) is introduced so that it extends along the entire length of the container.
